# EUROPEAN PATENT APPLICATION

(11) **EP 3 416 028 A1**
(43) Date of publication of application: **19.12.2018**
(21) Application number: 18174041.6
(22) Date of filing: 24.05.2018
(51) Int. Cl.: G06F 3/01

(54) **GENERATING HAPTIC EFFECT FOR A WEARABLE ELECTRONIC DEVICE BASED ON TIGHTNESS LEVEL**

(30) Priority: 13.06.2017 US 201715621182
(71) Applicant: Immersion Corporation, San Jose, CA 95134 (US)
(72) Inventor: RIHN, William S., San Jose, CA California 95124 (US)
(74) Representative: Hofstetter, Schurack & Partner

(57) **Abstract**

A wearable electronic device having a device body, a sensor, a haptic actuator, and a control unit is presented. The device body is wearable at different tightness levels. The sensor is configured, when the device body is being worn, to measure a parameter indicative of a tightness level by which the device body is being worn. The control unit is configured, when the device body is being worn, to receive from the sensor a measurement of the parameter indicative of the tightness level by which the device body is being worn. The control unit is further configured to determine the tightness level based on the measurement. When there is a haptic effect to be output, the control unit is configured to determine, based on the tightness level by which the device body is being worn, an intensity, duration, or frequency of a haptic driving signal for generating the haptic effect.

## Description

### FIELD OF THE INVENTION

The present invention is directed to generating a haptic effect for a wearable electronic device based on a tightness level by which the wearable electronic device is being worn, and has application in user interfaces, gaming, wearable devices, and consumer electronics.

### BACKGROUND

As electronic user interface systems become more prevalent, the quality of the interfaces through which humans interact with these systems is becoming increasingly important. Haptic feedback, or more generally haptic effects, can improve the quality of the interfaces by providing cues to users, providing alerts of specific events, or providing realistic feedback to create greater sensory immersion within a virtual environment. Examples of haptic effects include kinesthetic haptic effects (such as active and resistive force feedback), vibrotactile haptic effects, and electrostatic friction haptic effects.

### SUMMARY

The following detailed description is merely exemplary in nature and is not intended to limit the invention or the application and uses of the invention. Furthermore, there is no intention to be bound by any expressed or implied theory presented in the preceding technical field, background, brief summary or the following detailed description.

One aspect of the embodiments herein relate to a wearable electronic device that comprises a device body, a sensor, a haptic actuator, and a control unit. The device body is wearable at different tightness levels. The sensor is disposed on or within the device body, and is configured, when the device body is being worn, to measure a parameter indicative of a tightness level by which the device body is being worn. The haptic actuator is disposed on or within the device body. The control unit is disposed on or within the device body and is in communication with the sensor and with the haptic actuator. The control unit is configured, when the device body is being worn, to receive from the sensor a measurement of the parameter indicative of the tightness level by which the device body is being worn. The control unit is further configured to determine the tightness level based on the measurement from the sensor, to determine that a haptic effect is to be output at the device body, to determine, based on the tightness level by which the device body is being worn, an intensity, duration, or frequency of a haptic driving signal for generating the haptic effect. The control unit is further configured to activate the haptic actuator with the haptic driving signal to output the haptic effect, the haptic driving signal having the intensity, duration, or frequency that is determined.

In an embodiment, at least a first portion of the device body is configured to fit around or substantially fit around a second portion of a user, wherein the sensor is at least one of a strain sensor and a pressure sensor, and wherein the parameter measured by the sensor is at least one of a strain in the first portion of the device body and a pressure experienced by the first portion of the device body.

In an embodiment, the first portion of the device body comprises a strap, the device body further comprising a housing that is coupled to the strap, wherein the housing houses the control unit, and wherein the housing and the strap together fit around the second portion of the user when the device body is being worn.

In an embodiment, at least a first portion of the device body is configured to fit around or substantially fit around a second portion of a user, wherein the sensor is disposed on a surface of the first portion of the device body and is at least one of a temperature sensor, a heart or pulse rate sensor, and a depth of field sensor, and wherein the parameter measured by the sensor is at least one of a strength of a heart rate signal or of a pulse rate signal, a temperature, a depth of field of another surface facing the sensor, and an amount of skin contact with the first portion.

In an embodiment, the control unit is configured to determine the at least one of the intensity, duration, or frequency of the haptic driving signal by: determining i) whether the tightness level by which the device body is being worn is within a defined range of tightness levels, ii) whether the tightness level is above the defined range, or iii) whether the tightness level is below the defined range; in response to a determination that the tightness level is within the defined range, determine the at least one of the intensity, duration, or frequency as having a defined first level. The control unit is further configured, in response to a determination that the tightness level is below the defined range, determining the at least one of the intensity, duration, or frequency as having a second level that has a higher intensity, longer duration, or lower frequency than that of the first level. The control unit is further configured, in response to a determination that the tightness level is above the defined range, determine the at least one of the intensity, duration, or frequency as having a third level that has a lower intensity, shorter duration, or higher frequency than that of the first level.

In an embodiment, the sensor is configured to measure the parameter even when the device body is not being worn, wherein the control unit is configured to determine whether the device body is being worn by determining, based on the parameter measured by the sensor, whether the tightness level is at or above a defined tightness threshold, the defined tightness threshold being below the defined range of tightness levels, and wherein the control unit determines that the haptic effect is to be output by the wearable electronic device only if the control unit has determined that the device body is being worn.

In an embodiment, the wearable electronic device further comprises a storage device storing a profile that defines a plurality of defined ranges of tightness levels, and wherein the control unit is configured to identify a material that forms at least a portion of the device body, and is configured to select the defined range of tightness levels from among the plurality of defined ranges based on the material that is identified.

In an embodiment, the sensor is a first sensor, the wearable electronic device further comprising a second sensor that is a skin contact sensor configured to detect whether the device body is experiencing skin contact. The control unit is configured in response to a determination that the device body is being worn and is experiencing skin contact, to select a stored level of intensity, duration, or frequency associated with skin contact as being the defined first level, and in response to a determination that the device body is being worn and is not experiencing skin contact, to select a stored level of intensity, duration, or frequency associated with clothing contact as being the defined first level, wherein the stored level of intensity, duration, or frequency associated with clothing contact has a higher intensity, longer duration, or lower frequency than that of the stored level of intensity, duration, or frequency associated with skin contact.

In an embodiment, the control unit is further configured, in response to a determination that the tightness level is within the defined range, to cause the haptic driving signal to have a first level of complexity by generating the haptic driving signal with a first number of pulses and a first duration between consecutive pulses, and in response to a determination that the tightness level is below the defined range, to cause the haptic driving signal to have a second level of complexity lower than the first level by generating the haptic driving signal with a second number of pulses and a second duration between consecutive pulses, wherein the second number is less than the first number, and the second duration is longer than the first duration.

In an embodiment, the wearable electronic device further comprises a storage device storing a profile that associates a plurality of levels of at least one of intensity, duration, or frequency of haptic driving signals with respective materials of device bodies, and is configured to determine the intensity, duration, or frequency of the haptic driving signal by identifying a material that forms at least a portion of the device body, and selecting one of the plurality of levels of intensity, duration, or frequency based on the material that is identified.

In an embodiment, the plurality of materials includes a first material having a first rigidity level and a second material having a second rigidity level higher than the first rigidity level, and wherein the first material is associated with a first level of intensity, duration, or frequency that has a higher intensity, longer duration, or lower frequency than that of a second level of intensity, duration, or frequency associated with the second material.

In an embodiment, the haptic actuator is one of a plurality of haptic actuators disposed on the device body, and wherein the control unit is configured to determine that the device body is being worn, and to determine which one or more haptic actuators of the plurality of haptic actuators are in contact with a user, and is configured to select only from among the one or more haptic actuators that are determined to be in contact with the user to output the haptic effect.

In an embodiment, the wearable electronic device further comprises a storage device that defines a first level of at least one of intensity, duration, or frequency at which to activate the haptic actuator, wherein the control unit is further configured to calibrate the haptic actuator by activating the haptic actuator with the first level to output a second haptic effect, determining whether a measured intensity of the second haptic effect is outside a defined range of haptic effect intensities, and in response to a determination that the measured intensity is outside the defined range, adjust the first level of the at least one of intensity, duration, or frequency, and store the adjusted first level as the defined first level in the storage device.

One aspect of the embodiments herein relate to a method of generating one or more haptic effects for a wearable electronic device having a device body that is wearable at different tightness levels. The method is performed by a processor and comprises receiving, from a sensor disposed on or within the device body, a measurement of a parameter indicative of a tightness level by which the device body is being worn; determining the tightness level based on the measurement from the sensor; determining that a haptic effect is to be output at the device body; determining, based on the tightness level by which the device body is being worn, an intensity, duration, or frequency of a haptic driving signal for generating the haptic effect; and activating a haptic actuator disposed on or within the device body with the haptic driving signal to output the haptic effect, the haptic driving signal having the intensity, duration, or frequency that is determined.

In an embodiment, the step of determining the at least one of the intensity, duration, or frequency of the haptic driving signal comprises: determining i) whether the tightness level by which the device body is being worn is within a defined range of tightness levels, ii) whether the tightness level is above the defined range, or iii) whether the tightness level is below the defined range, wherein the defined range of tightness levels is associated with a defined first level of intensity, duration, and frequency for the haptic driving signal.

In an embodiment, the tightness level is determined to be below the defined range, the method further comprising determining the at least one of the intensity, duration, or frequency as having a second level that has a higher intensity, longer duration, or lower frequency than that of the first level.

In an embodiment, the tightness level is determined to be above the defined range, the method further comprising determining the at least one of the intensity, duration, or frequency as having a third level that has a lower intensity, shorter duration, or higher frequency than that of the first level.

In an embodiment, the method further comprises determining that the device body is being worn by determining, based on the parameter measured by the sensor, that the tightness level is at or above a defined tightness threshold, wherein the defined tightness threshold is below the defined range of tightness levels, and wherein the step of determining that the device body is being worn is performed before the step of determining that the haptic effect is to be output at the device body.

In an embodiment, the step of determining the intensity, duration, or frequency of the haptic driving signal is based on a material that forms at least a portion of the device body.

In an embodiment, the method further comprises detecting whether the device body is experiencing skin contact or is instead receiving clothing contact, and wherein the step of determining the intensity, duration, or frequency of the haptic driving signal is based on whether the device body is experiencing skin contact or is instead receiving clothing contact.

Features, objects, and advantages of embodiments hereof will become apparent to those skilled in the art by reading the following detailed description where references will be made to the appended figures.

### BRIEF DESCRIPTION OF THE DRAWINGS

The foregoing and other features and advantages of the invention will be apparent from the following description of embodiments hereof as illustrated in the accompanying drawings. The accompanying drawings, which are incorporated herein and form a part of the specification, further serve to explain the principles of the invention and to enable a person skilled in the pertinent art to make and use the invention. The drawings are not to scale.
FIGS. 1A and 1B depict perspective views of various wearable electronic devices that are each capable of outputting a haptic effect, according to an embodiment hereof.
FIG. 2A illustrates a view of a device body of a wearable electronic watch, according to an embodiment hereof.
FIG. 2B illustrates a block diagram of a wearable electronic watch, according to an embodiment hereof.
FIGS. 3A and 3B illustrate an increase in a tightness level by which a wearable electronic device is worn, and an adjustment of a haptic driving signal based on the increase in the tightness level, according to an embodiment hereof.
FIGS. 4A and 4B illustrate an increase in a tightness level by which a wearable electronic device is worn, and an adjustment of a haptic driving signal based on the increase in the tightness level, according to an embodiment hereof.
FIGS. 5-10 illustrate flow diagrams of various steps for generating a haptic effect for a wearable electronic device, according to an embodiment hereof.

### DETAILED DESCRIPTION

The following detailed description is merely exemplary in nature and is not intended to limit the invention or the application and uses of the invention. Furthermore, there is no intention to be bound by any expressed or implied theory presented in the preceding technical field, background, brief summary or the following detailed description.

Embodiments hereof relate to generating a haptic effect (e.g., a vibrotactile haptic effect) for a wearable electronic device (e.g., a smart watch, a fitness band, a smart glove, or a gaming vest) for different levels of tightness (i.e., different tightness levels) by which the wearable electronic device is worn. More specifically, the wearable electronic device may have a device body that is wearable at different tightness levels. For instance, the wearable electronic device may be a wearable electronic watch (e.g., an Apple Watch®), and the device body may include a combination of a watch housing and a strap that together make the device body wearable. The tightness level at which the electronic watch is worn (e.g., loosely or tightly) may change for a user if the user adjusts a size of a loop formed by the strap, and/or if the user slides the device body up his or her wrist toward the forearm (which may have a larger cross section than at the wrist). In another example, the tightness level at which the electronic watch is worn may be different for two different users, whose wrist sizes may differ. Embodiments herein relate to compensating for different tightness levels by which the device body is being worn when generating a haptic effect for the wearable electronic device. Doing so may allow the wearable electronic device to output haptic effects that attempt to impart generally similar sensations for different tightness levels at which the wearable electronic device is worn (e.g., for different body sizes). This compensation may involve, e.g., varying a haptic driving signal that is output by the wearable electronic device based on whether its device body is being worn tightly or being worn loosely. In an embodiment, this compensation may aid a designer or other author of a haptic effect. For instance, an author may define a haptic driving signal with a particular tightness level in mind. Without compensating the haptic driving signal for other tightness levels, the authored haptic effect may be perceived in unintended or sub-optimal ways at the other tightness levels. As an example, the authored haptic effect may be perceived as being too weak when the device body is being worn too loosely (i.e., at a tightness level that is too low), and may be perceived as too strong when the device body is worn too tightly (i.e., at a tightness level that is too high).

In an embodiment, the wearable electronic device may compensate for different tightness levels by controlling a haptic driving signal, such as an intensity (e.g., amplitude), frequency, and/or or duration thereof, based on a tightness level by which a device body of a wearable electronic device is being worn. When a wearable electronic device is loosely worn, the haptic effect may be generated with a haptic driving signal that has a level of higher intensity, lower frequency, and/or lower duration compared to, e.g., a defined level of intensity, frequency, or duration for an authored haptic effect. When the wearable electronic device is tightly worn, the haptic effect may be generated with haptic driving signal that has a level of lower intensity, higher frequency, and/or lower duration compared to, e.g., the defined intensity, frequency, or duration for the authored haptic effect.

In an embodiment, the wearable electronic device may compensate for different tightness levels by controlling its level of complexity. The complexity of a haptic effect may be associated with parameters such as how many pulses are in the haptic effect, a duration between consecutive pulses, and uniformity of the pulses. A more complex haptic effect may have more pulses, pulses that are spaced closer together, and/or pulses with a high degree of variance in, e.g., pulse widths. A more complex haptic effect may be used to, e.g., simulate a more complex surface texture. When a wearable electronic device is loosely worn, a high degree of complexity for a haptic effect may be unnecessary because it can be difficult to perceive anyway, and may use more power than a less complex haptic effect. Thus, when the wearable electronic device is loosely worn, it may control its haptic effect to have a lower level of complexity compared to a defined level of complexity for an authored haptic effect.

In an embodiment, a wearable electronic device may compensate for a material of its device body when generating a haptic effect (e.g., whether an electronic watch has a leather strap or a metal strap (also referred to as a metal band)). The material may have a parameter, such as rigidity, that may affect the determination of when a device body is being worn too tightly or too loosely, and/or may affect how a haptic driving signal is adjusted (e.g., modulated) when the device body is considered to be worn too tightly or too loosely.

In an embodiment, the wearable electronic device may compensate for whether its device body is being worn over clothing, as opposed to being in direct contact with a user's skin. If the wearable electronic device is being worn over the user's clothing, the haptic driving signal may, e.g., have an increased intensity, increased duration, and/or decreased frequency.

In an embodiment, the wearable electronic device may be configured to determine whether its device body is being worn, and to disable (i.e., mute) haptic effects when the device body is not being worn.

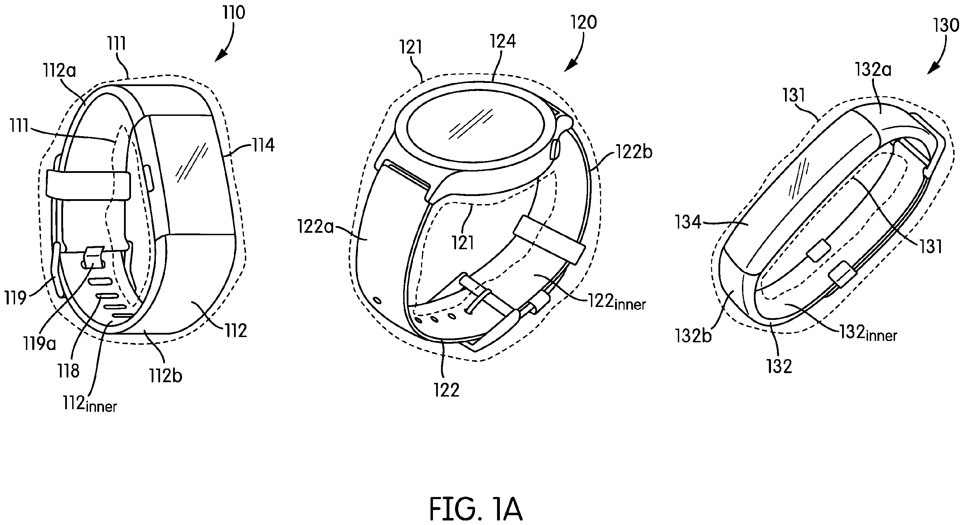

FIG. 1A illustrates various wearable electronic devices 110, 120, and 130 that are wearable around a user's wrist. Wearable electronic devices 110, 120, 130 may all be an activity tracker, while wearable electronic devices 110 and 120 may more specifically be an electronic watch (e.g., a smart watch). Each of the wearable electronic devices 110, 120, 130 may have a respective device body 111, 121, 131 that is wearable at different tightness levels. In the embodiment of FIG. 1A, the device body 111 may comprise a strap 112 and a housing 114 coupled to the strap 112. The housing 114 may house electronic components such as a control unit and display of the wearable electronic device 110. In FIG. 1A, the strap 112 may be formed from two strips 112a, 112b of material (e.g., two plastic strips) that are detachable from and attachable to each other (e.g., via buckle 119). In another embodiment, a strap may be formed from a single strip (also referred to as a band, such as a metal band) that is attached to two opposite edges of a watch housing. The strip may be tightened or loosened via, e.g., a butterfly clasp. As illustrated in FIG. 1A, the housing 114 and the strap 112 may together be configured to fit around a portion (e.g., a wrist) of a user when the device body 111 is being worn.

As further depicted in FIG. 1A, device body 121 may comprise a strap 122 that is coupled to a housing 124 of electronic components (e.g., a touch screen, a speaker, a wireless communication circuit, a battery, and a general purpose processor). Like with strap 112, the strap 122 may include two strips 122a, 122b of material (e.g., leather) that are detachable from and attachable to each other via a buckle. The housing 124 and the strap 122 may together be configured to fit around a user's wrist when the device body 121 is being worn. Similarly, device body 131 may comprise a strap 132 and a housing 134 coupled to the strap 132. Like with strap 112 and 132, the strap 132 may include two strips 132a, 132b of material that are detachable from and attachable to each other. The housing 134 may house electronic components such as a motion sensor and a wireless communication circuit. The housing 134 and the strap 132 (having a pair of strips 132a, 132b) may together be configured to fit around a user's wrist when the device body 134 is being worn. In an embodiment, when one of the wearable electronic devices 110, 120, or 130 is worn, a respective inner surface 112ᵢₙₙₑᵣ, 122ᵢₙₙₑᵣ, or 132ᵢₙₙₑᵣ of the straps 111, 121, and 131, respectively, may press against the user's wrist.

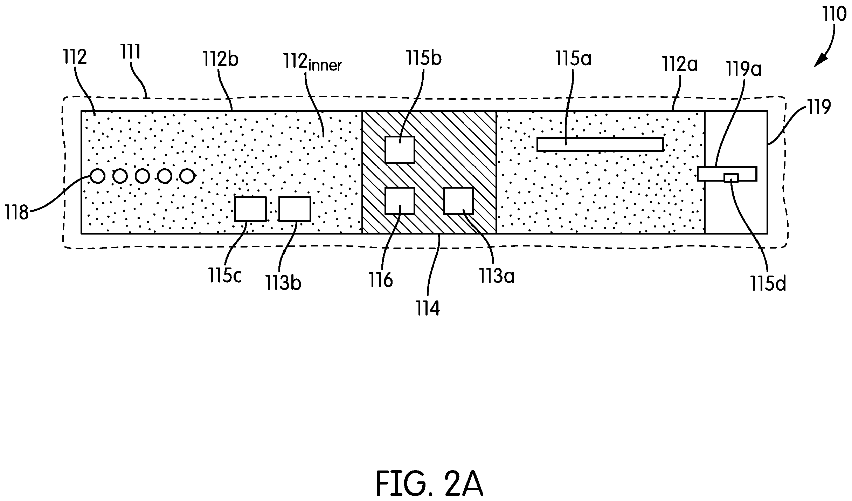

In an embodiment, each device body 111, 121, and 131 may be wearable at different tightness levels. For instance, a tightness level at which the device body 111 is worn may be adjusted with a buckle 119 and an array of holes 118, which are illustrated in FIG. 1A. The buckle 119 may be a pin buckle (as depicted in FIG. 2A), a deployment clasp buckle, or any other type of buckle. The buckle 119 may comprise a tongue 119a that can be inserted into one of the array of holes 118. The selection of which of the holes 118 the tongue 119a is inserted into may affect the tightness level by which the device body 111 is worn. In an embodiment, the tightness level at which the device body 111 is worn may be adjusted by being slid up or down a user's wrist. For instance, a user's wrist may widen as the device body 111 is slid in a direction from the user's hand toward the user's elbow. As the device body 111 is slid up along the user's wrist in that direction, the tightness level by which the device body 111 is being worn may increase.

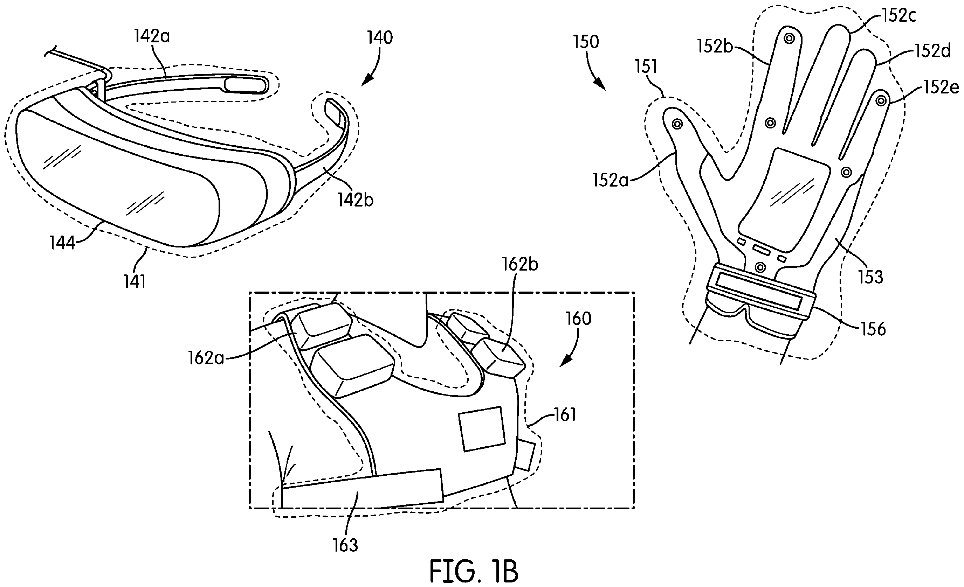

FIG. 1B illustrates other examples of wearable electronic devices, including a virtual reality (VR) head-mounted display (HMD) 140, a haptic-enabled glove 150, and a haptic-enabled gaming vest 150. In an embodiment, the wearable electronic device 140 may have a device body 141 that is wearable at different tightness levels. The device body 141 may fit substantially around a portion of a user (e.g., the user's head) when the device body 141 is being worn. The device body 141 may comprise a housing 144 and a pair of arms (also referred to as temples) 142a, 142b extending from the housing 144. The housing 144 may house electronic components of the wearable electronic device 140, such as a display device, one or more motion sensors (e.g., an accelerometer and a gyroscope), a wireless communication unit for communicating with a host computer, and a speaker. The housing 144 and the pair of arms 142a, 142b may together fit substantially around the user's head. As illustrated in FIG. 1B, the housing 144 and the pair of arms 142a, 142b do not need to completely fit around a user's head, but may fit substantially around the user's head so as to achieve a level of tension or friction with the user's head that keeps the device body 141 on the user's head (or other body portion). In an embodiment, the tightness level by which the device body 141 fits substantially around the user's head may depend on a size of the user's head.

In an embodiment, the haptic-enabled glove 150 may comprise a device body 151 that is wearable around a user's hand at different tightness levels. The device body 151 may include a plurality of finger sheaths 152a-152e that fit around a respective finger of a user's hand, a cuff 156 that fits around the user's wrist, and a palm portion 153 that covers the palm and back of the user's hand. In an embodiment, the tightness level by which the device body 151 is worn may depend on the size of a user's hand. In an embodiment, if the cuff 156 is adjustable, the tightness level by which the body 151 is worn may depend on a tightness level by which the cuff 156 fits around the user's wrist.

In an embodiment, the haptic-enabled gaming vest 160 may have a device body 161 that is wearable at different tightness levels. The device body 161 may include a pair of shoulder straps 162a, 162b and a chest portion 163 that fits around the chest and back of a user. In an embodiment, the tightness level by which the device body 161 is worn may depend on a shoulder or chest width of a user. In an embodiment, if the shoulder straps 162a, 162b and/or chest portion 163 are adjustable, the tightness level by which the device body 161 is worn may depend on how the straps 162a, 162b and/or the chest portion are adjusted.

FIG. 2A depicts a view of the wearable electronic device 110 (e.g., electronic watch) when it is not being worn, and further illustrates haptic actuators and sensors disposed on or within the wearable electronic device 110. FIG. 2A illustrates the pair of strips 112a, 112b of strap 112, and the housing 114 of the wearable electronic device 110. FIG. 2A further depicts a haptic actuator 113a disposed on or within the housing 114, and a haptic actuator 113b disposed on or within the strip 112b. In an embodiment, haptic actuator 113a and/or 113b may be configured to generate a vibrotactile haptic effect. In an embodiment, haptic actuator 113a and/or 113b may be configured to generate a more general deformation-based haptic effect, a kinesthetic haptic effect, an electrostatic haptic effect, any other type of haptic effect, or any combination thereof. Examples of the haptic actuators 113a, 113b include a smart-material-based haptic actuator (e.g., piezoelectric actuator, shape memory alloy actuator), a linear resonant actuator, a solenoid resonant actuator, a static electrostatic haptic actuator, or any other type of haptic actuator.

FIG. 2A further illustrates sensors 115a-115d, which are disposed on or within the device body 111 and configured, when the device body 111 is being worn, to measure a parameter indicative of a tightness level by which the device body 111 is being worn. In an embodiment, sensor 115a may be a strain sensor disposed on the strap 112 or embedded within the strap 112. The sensor 115a may, for instance, be aligned along a length of the strap 112. The sensor 115a may be configured to measure a parameter such as strain in the device body 111, and more specifically in the strap 112. The strain may be indicative of a level to which the strap 112 is stretched, which may further indicate how tightly the device body 111 fits around a user's wrist.

In an embodiment, sensor 115b may be disposed on a rear surface of housing 114, and may be a temperature sensor, a heart rate sensor, a pulse rate sensor, a depth of field sensor, or any combination thereof. The sensor 115b may be configured to measure a parameter such as a strength of a heart rate signal or of a pulse rate signal, a temperature, or a depth of field of a surface nearest the device body. Such a parameter may be indicative of a tightness level by which device body 111 fits around a user's wrist. For instance, if the device body 111 were loosely worn, housing 114 and the sensor 115b of the device may be more lightly pressed against the user's wrist, if at all. As a result, the strength of any heart rate signal or pulse rate signal may be low, and the temperature immediately around the sensor 115b may be closer to ambient temperature than an average human body temperature. Thus, a measurement which yields a low strength of a heart rate signal or a pulse rate signal, or which yields a temperature which is within a threshold amount of an ambient temperature (e.g., 20 degrees Celsius +/- 10 degrees), may indicate that the tightness level by which the device body 111 fits around the user's wrist is low. If the device body 111 were tightly worn, housing 114 and the sensor 115b of the device may be more tightly pressed against the user's wrist. As a result, the sensor 115b may output a measurement which indicates a strong heart rate signal, a strong pulse rate signal, or a temperature which is within a threshold amount of an average body temperature (37 degrees Celsius +/- 5 degrees). Such a measurement may indicate that the tightness level by which the device body fits around the user's wrist is high.

In an embodiment, sensor 115c may be a pressure sensor disposed on or just beneath the inner surface 112ᵢₙₙₑᵣ of the strap 112. The sensor 115c may be configured to measure a pressure experienced by the device body 111, such as a pressure received by the strap 112b from a user's wrist pressing against the strap 112b when the device body 111 is being worn. A high amount of pressure being exerted on the strap 112b may indicate that the device body 111 fits around the user's wrist with a high level of tightness. A low amount of pressure being exerted on the strap 112b may indicate that the device body 111 fits around the user's wrist with a low tightness level.

In an embodiment, sensor 115d may be a pressure sensor disposed on or contained within the buckle 119 of the wearable electronic device 110. As discussed above, the buckle 119 may be a pin buckle, as illustrated in FIG. 2A, a deployment clasp buckle, or any other type of buckle. The sensor 115d may be configured to sense pressure on the buckle 119 or a component thereof, which may indicate a tightness level by which the device body 111 fits around the user's wrist. For instance, as the tongue 119a is inserted into a hole of strip 112b to keep the two strips 112a, 112b attached, the strip 112b may exert pressure on the tongue 119a during the wearing of the device 110. The pressure on the tongue 119a may be affected by which of the holes 118 it is inserted into, and/or how high up a user's wrist the device body 111 is being worn. When the device body 111 of the wearable electronic device 110 is being tightly worn, more pressure may be experienced by the tongue 119a. When the wearable electronic device 110 is being loosely worn, less pressure may be experienced by the tongue 119a. Thus, sensor 115d may be disposed on the tongue 119a to measure a pressure being exerted on the tongue 119a by a portion of the strip 112b surrounding the hole into which the tongue 119a is inserted. This pressure may be indicative of a tightness level by which the device body 111 fits around a user's wrist. In an embodiment, the buckle 119 may contain a haptic actuator.

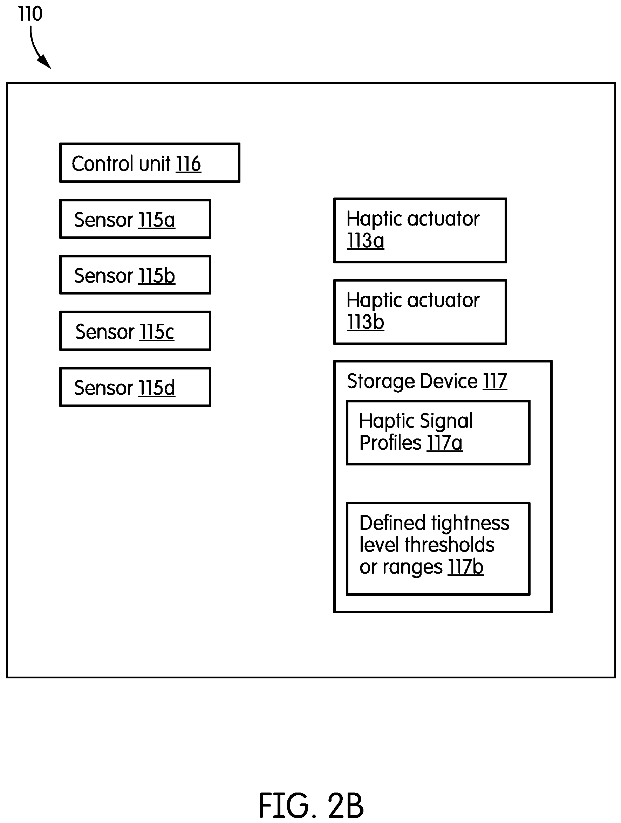

FIG. 2B depicts a block diagram of example components of a wearable electronic device 110. The example components include a control unit 116, haptic actuators 113a, 113b, sensors 115a-115d, and a storage device 117. The control unit 116 may be in communication with the haptic actuators 113a, 113b and with the sensors 115a-115d. The haptic control unit 116 may further be configured to provide a haptic driving signal to at least one of the haptic actuators 113a, 113b in order to activate the haptic actuator. In an embodiment, the control unit may be configured to determine, based on the measured parameter from one or more of the sensors 115a-115d, a tightness level by which the device body 111 of the wearable electronic device 110 fits around or substantially around a portion of a user. As discussed in more detail below, when the device body 111 is being worn, the control unit 116 may be configured to receive a measurement from a sensor of a measurement indicative of the tightness level by which the device body is being worn, and determine the tightness level based on the sensor measurement. The control unit may further be configured to determine that a haptic effect is to be output at the device body 111, and to determine, based on the tightness level by which the device body 111 fits around the user's wrist, a level of intensity, duration, or frequency of the haptic driving signal provided to a haptic actuator for generating a haptic effect. The haptic actuator may then be activated with the determined haptic driving signal. In an embodiment, the control unit 116 may be implemented as a microprocessor that is configured to execute one or more non-transitory computer-readable instructions stored on a memory. In an embodiment, the control unit 116 may be implemented as a preprogrammed logic circuit, such as a field programmable gate array (FPGA).

In an embodiment, the storage device 117 may store haptic signal profiles 117a, which may define various haptic driving signals (e.g., for various authored haptic driving signals). Each stored haptic signal profile 117a may include a complete waveform for a haptic driving signal, or may include an intensity value, frequency value, and/or duration value of a haptic driving signal. In an embodiment, the storage device 117 may store a plurality of defined tightness level thresholds or ranges 117b. These tightness level thresholds or ranges may be used to determine whether to adjust a defined haptic driving signal, as discussed below in more detail. In an embodiment, a defined haptic driving signal may be adjusted by modulating (e.g., multiplying) the signal by a factor, e.g., 75% or 150%.

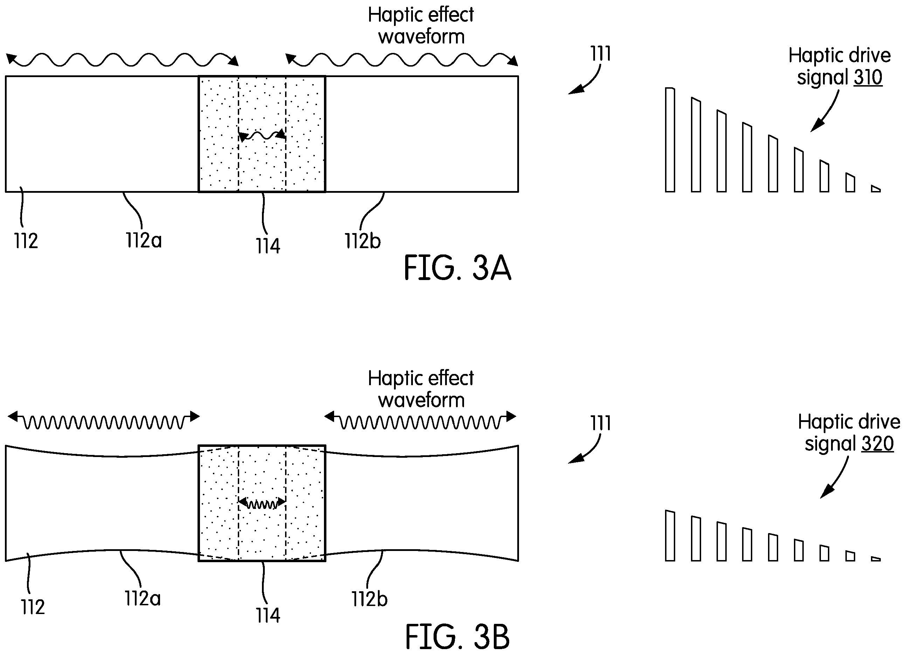

FIGS. 3A and 3B illustrate a technique in which, as the tightness level for the device body 111 increases, the intensity of a haptic driving signal may generally decrease, and its frequency may generally increase. More specifically, FIG. 3A represents the device body 111 being worn at a first tightness level. For this first tightness level, the control unit 116 of FIG. 2B may generate a haptic driving signal 310 and provide the signal to haptic actuator 113 a and/or 113b of FIG. 2B to cause a haptic effect to be output at the device body 111. In an embodiment, the haptic driving signal 310 may be a signal defined in the haptic signal profiles 117a of FIG. 2B. The defined haptic driving signal may have been designed to achieve a specific haptic effect. The defined haptic driving signal may be referred to as an authored driving signal, and the corresponding haptic effect may be referred to as an authored haptic effect.

FIG. 3B represents the device body 111 being worn at a second tightness level that is higher than the first tightness level. In this scenario, the pair of strips 112a and 112b may be stretched to be more taut than in FIG. 3A, and the device body 111 may be pressed more tightly against the user's wrist than in FIG. 3A. As the device body 111 of the wearable electronic device 110 is worn at increasing tightness levels, a user's perception of or sensitivity to an authored haptic effect may increase. This increased perception may arise from the device body 111 being closer to the user's body, the device body 111 being pressed more tightly against the user's body, and/or portions of the device body 111 being more taut. These factors allow, for example, vibrations of a vibrotactile haptic effect to propagate more easily from the device body 111 to the user's body, and with less attenuation, thus enhancing a user's perception of the vibrotactile haptic effect. An author of a haptic effect may not have anticipated, however, how the haptic effect will be perceived at a broad range of tightness levels. For instance, the author may have selected a signal intensity and frequency based on a tightness level at which the author wears a wearable electronic device. When another user wears the device body of the wearable electronic device more loosely, the user may perceive the haptic effect to be weaker than what the author intended. When yet another user wears the device body more tightly, the user may perceive the haptic effect to be stronger than what the author intended. In an embodiment, when the perception of the haptic effect becomes too strong, it may become unpleasant or distracting.

FIG. 3B illustrates adjusting an authored haptic driving signal based on an increase in tightness level at which the device body 111 is being worn. In FIG. 3B, the device body 111 may be worn at a tightness level that exceeds a baseline tightness level (e.g., by 50%). The baseline tightness level may be, e.g., depicted in FIG. 3A. At the tightness level represented in FIG. 3B, the intensity of the haptic driving signal 320 may be decreased compared to that of an authored haptic driving signal 310, and/or the frequency of the haptic driving signal 320 may be increased compared to that of the authored haptic driving signal 310. The frequency may be increased because, e.g., high-frequency components of a vibration or other type of haptic effect may experience a greater level of attenuation. In an embodiment, the duration of the haptic driving signal 320 may be decreased compared to that of the authored haptic driving signal 310 (e.g., decreased by 50%).

In an embodiment, the haptic driving signal 320 may be an adjusted signal derived from haptic driving signal 310. For instance, haptic driving signal 320 may be generated by retrieving the authored haptic driving signal 310 from the haptic signal profiles 117a, and modifying the intensity and/or frequency of the signal 310 to yield the signal 320. In an embodiment, the haptic driving signal 320 may be selected from a plurality of haptic driving signals, including signals 310 and 320, stored in the haptic signal profiles 117a of storage device 117. For instance, the plurality of stored haptic driving signals may be associated in a table with different respective tightness levels, and haptic driving signal 320 may be selected based on the device body 111 being worn at an associated tightness level.

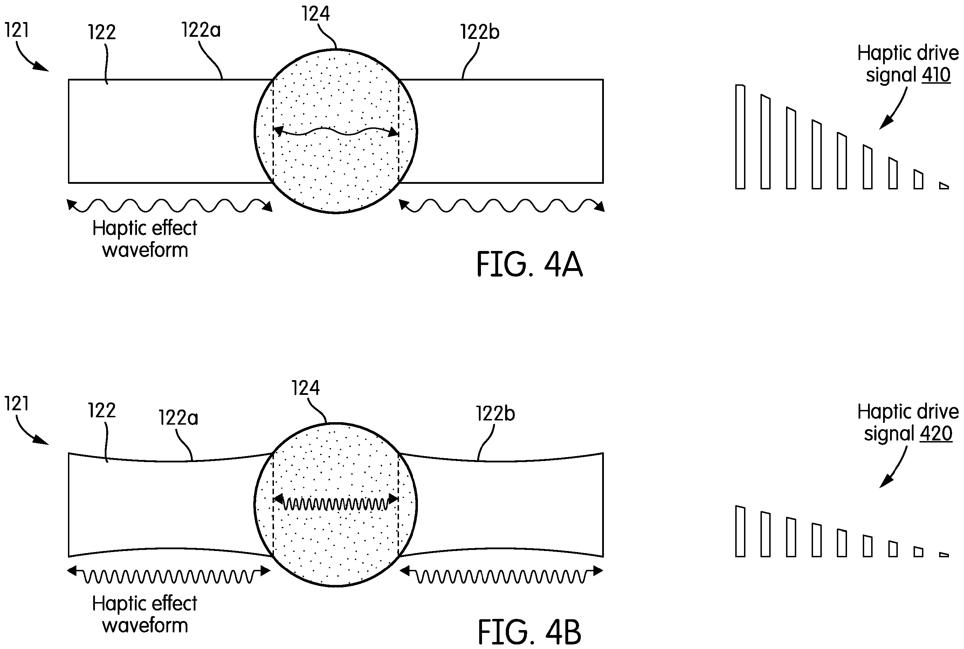

FIGS. 4A and 4B similarly illustrate selecting or adjusting a haptic driving signal based on a tightness level at which device body 121 of wearable electronic device 120 is worn. In an embodiment, FIG. 4A may represent the device body 121 being worn at a baseline level of tightness, while FIG. 4B may represent the device body 121 being worn at an increased level of tightness. FIG. 4B illustrates the increased tightness level resulting in a haptic driving signal 420 with a lower intensity and/or higher frequency compared to the haptic driving signal 410 used for the baseline level of tightness.

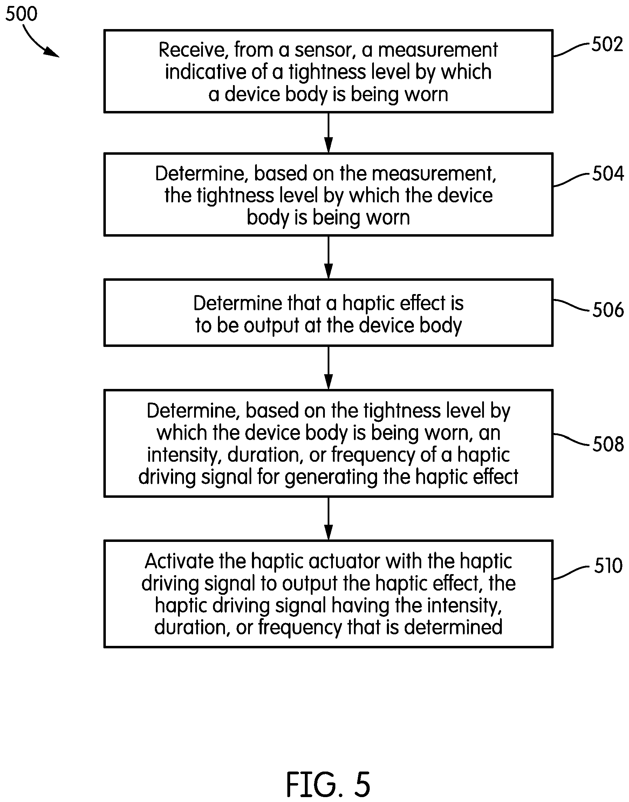

FIG. 5 provides a flow diagram that illustrates an example method 500 for outputting a haptic effect that is based on a tightness level at which a wearable electronic device (e.g., device 110) is worn. In an embodiment, method 500 is performed by a control unit (e.g., control unit 116) of a wearable electronic device. In an embodiment, the steps illustrated in FIG. 5 may be performed while the wearable electronic device is being worn. However, additional figures in this disclosure present other steps that further determine whether the device body is in fact being worn. In an embodiment, method 500 begins at step 502, in which the control unit receives, from a sensor, a measurement indicative of a tightness level by which a device body of the wearable electronic device (e.g., device body 111) is being worn. In an embodiment, the sensor may be a strain sensor, a pressure sensor, a temperature sensor, a heart rate sensor, a pulse rate sensor, a depth of field sensor, or any combination thereof, as described above. In an embodiment, the measurement from the sensor may be a strain in the device body, a pressure experienced by the device body from a user's body, a strength of a heart rate signal or a pulse rate signal, a temperature, or a depth of field of a surface nearest the device body, as also discussed above.

In step 504, the control unit may determine, based on the measurement from the sensor, the tightness level by which the device body is being worn. In an embodiment, the tightness level may be represented by a dimensionless value that is on a scale of, e.g., 0-10, with a lower bound of the scale being a minimum defined tightness level, and an upper bound of the scale being a maximum defined tightness level. In an embodiment, the tightness level may be equal to or derived from by a strain, tension force, or pressure value.

In step 506, the control unit may determine that a haptic effect is to be output at the device body. In an embodiment, the haptic effect may be triggered by an event in an application. The application may be executing in the wearable electronic device (e.g., device 110) of the device body, in a computer in communication with the wearable electronic device (e.g., device 140), or any combination thereof. In an embodiment, the determination in step 506 may be a result of a request or command from the application for a haptic effect, or a result of a notification from the application that the event has occurred. In an embodiment, the haptic effect may be triggered by the tightness level measurement made in step 502. For instance, when a user of the wearable electronic device 110 pulls the strap 112 tight, a haptic effect in the form of a haptic detent may be triggered.

In step 508, the haptic control unit may determine, based on the tightness level by which the device body is being worn, an intensity, duration, or frequency of a haptic driving signal for generating the haptic effect. In step 510, the haptic control unit may activate the haptic actuator with the haptic driving signal to output the haptic effect, where the haptic driving signal has the determined intensity, duration, or frequency.

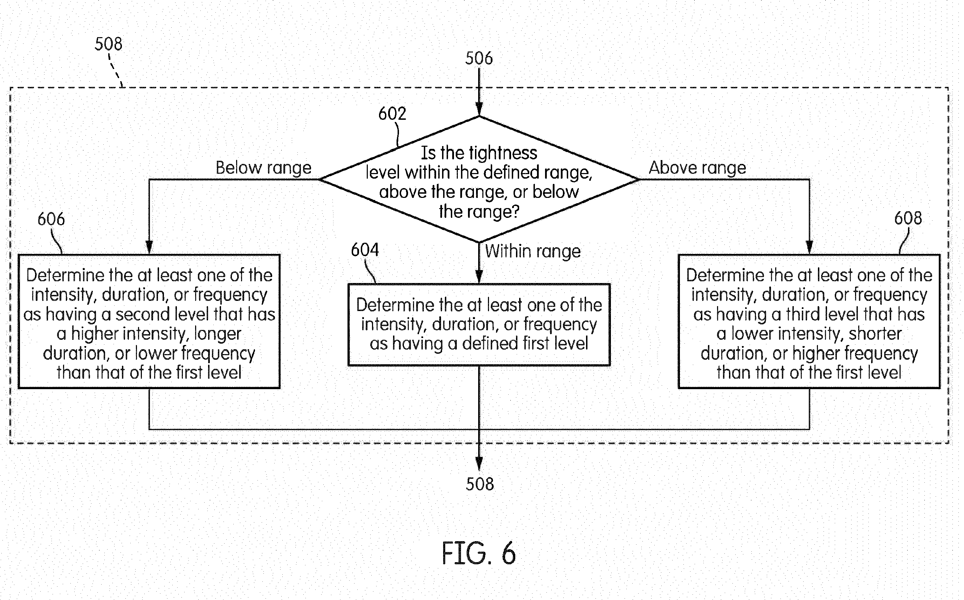

FIG. 6 illustrates an example of how step 508, the determination of the intensity, duration, or frequency, is performed. In this example, the determination involves steps 602-608. In step 602, the haptic control unit determines i) whether the tightness level by which the device body is being worn is within a defined range of tightness levels, ii) whether the tightness level is above the defined range, or iii) whether the tightness level is below the defined range. In an embodiment, the defined range may be a range of values for which an authored haptic effect was designed. For instance, while a wearable electronic device may be expected to encounter tightness levels from a value of 0 to 10 (or some other range of expected tightness levels), the authored haptic effect may have been designed and tested for a moderate tightness level of 5. In that instance, the defined range may be, e.g., from a tightness level of 4 to a tightness level of 6. The same defined range may be used across all models/types of a wearable electronic device, or, as discussed in more detail below, may be based on factors such as a material (e.g., leather or metal) of the device body. In an embodiment, the defined range may be one of the defined tightness ranges 117b in FIG. 2B. In an embodiment, the defined range may correspond with a high quality level of coupling to a user. When the tightness level is greater than this range or lower than this range, the tightness level may be considered to be at a low quality level of coupling to a user.

In response to a determination that the tightness level is within the defined range, the haptic control unit in step 604 may determine the at least one of the intensity, duration, or frequency as having a defined first level. In an embodiment, the defined first level may be a level defined in a haptic signal profile (e.g., profile 117a) used for defining authored haptic effects. In an embodiment, an authored haptic effect may be defined in terms of its haptic driving signal, such as an intensity, duration, and/or frequency, thereof. These values may be stored in the haptic signal profile.

In response to a determination that the tightness level is below the defined range, the haptic control unit may in step 606 determine the at least one of the intensity, duration, or frequency as having a second level that has a higher intensity, longer duration, or lower frequency than that of the first level. In an embodiment, the second level may calculated from the first level. In an embodiment, the second level may be selected from a plurality of levels based on its association (e.g., in haptic signal profile 117a) with the tightness level determined in step 504.

In response to a determination that the tightness level is above the defined range, the haptic control unit may in step 608 determine the at least one of the intensity, duration, or frequency as having a third level that has a lower intensity, shorter duration, or higher frequency than that of the first level.

In an embodiment, a complexity level of a haptic driving signal may be based on the determination of step 602. A more complex haptic driving signal may have, e.g., more pulses, shorter pulses, shorter interval between consecutive pulses, and/or more variation among pulses. When a wearable electronic device is worn too loosely, however, a more complex haptic effect may be harder to perceive. In such a situation, a less complex haptic effect can be generated. For instance, a haptic control unit may, in response to a determination that a tightness level by which a device body is being worn is within a defined range, cause a haptic driving signal to have a first level of complexity by generating the haptic driving signal with a first number of pulses and a first duration between consecutive pulses. In response to a determination that the tightness level is below the defined range, however, the control unit may cause the haptic driving signal to have a second level of complexity lower than that of the first level by generating the haptic driving signal with a second number of pulses and a second duration between consecutive pulses. In this example, the second number is first less than the first number, and the second duration is longer than the first duration.

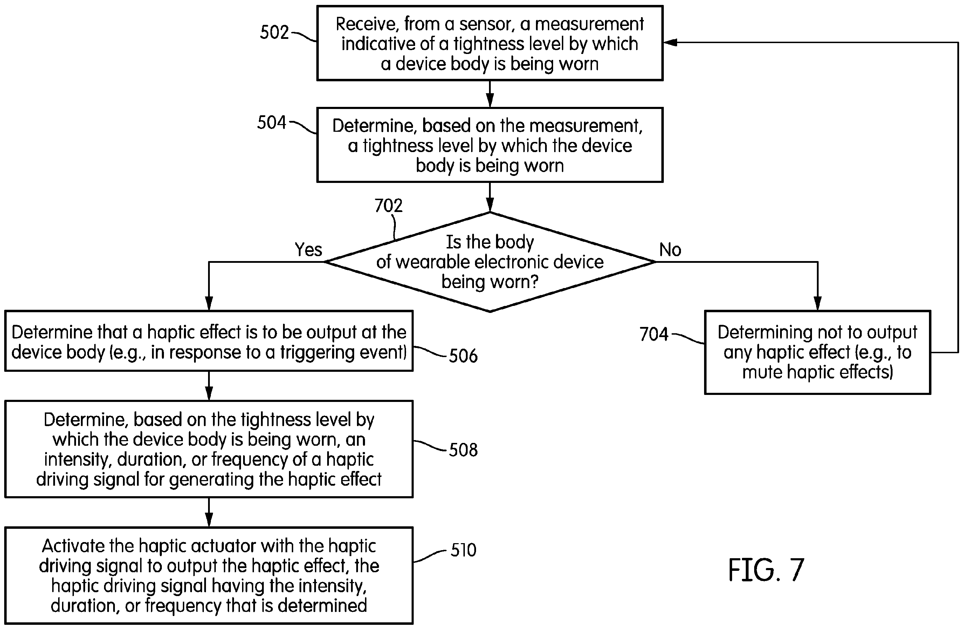

As discussed above, some of the steps (e.g., steps 506-508) in FIGS. 5 and 6 may be performed while a device body of a wearable electronic device is being worn. The sensor measurements and the determination of tightness levels in steps 502 and 504 may, however, be performed even when the device body is not being worn. FIG. 7 depicts a flow diagram of an embodiment in which the haptic control unit (or another device) checks that the device body is in fact being worn. Like in FIG. 5, the haptic control unit performs the step (502) of receiving, from a sensor, a measurement indicative of a tightness level by which a device body is being worn, and of determining, based on the measurement, a tightness level by which the device body is being worn. FIG. 7 further depicts a step 702, in which the haptic control unit determines whether the device body is being worn by determining, based on the parameter measured by the sensor, whether the tightness level is at or above a defined tightness threshold. The defined tightness threshold is generally below the defined range of tightness levels discussed above with respect to steps 602-608. For instance, an example defined range of 4-6 was provided for the above discussion of steps 602-608, while the defined tightness threshold for step 702 may have a value of, e.g., 0.5 or 1. The defined threshold tightness threshold may be, e.g., one of the stored defined tightness level thresholds or ranges 117b. The haptic control unit may determine (e.g., in step 506) that the haptic effect is to be output by the wearable electronic device only if the control unit has determined that the device body is being worn. Thus, in response to a determination to the device body of the wearable electronic device is not being worn, the haptic control unit may in step 704 mute haptic effects, or more generally determine not to output any haptic effect. The haptic effects may be muted until the haptic control unit determines that the device body is being worn.

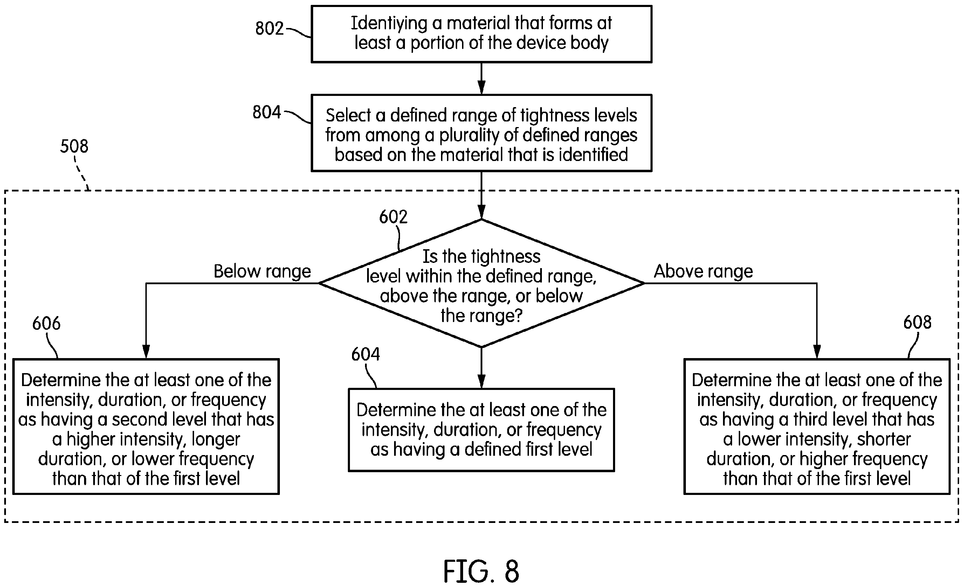

In an embodiment, as discussed above, the defined range used for step 602 may be used across all models/types of a wearable electronic device, or, as discussed in more detail below, may be based on factors such as a material of the device body. FIG. 8 depicts an example of this situation. The defined range may vary based on material of the device body because some materials may have more tolerance for a variations in tightness levels than other materials. In an embodiment, a storage device may store a plurality of defined ranges (e.g., 4.0-6.0 associated with a leather strap; 4.8-5.2 associated with a metal strap; 5.5-6.0 or 4.0-4.5 associated with some other material) and associate each defined range with a particular material (e.g., plastic, metal, leather, etc.). In an embodiment, the haptic control unit may, in step 802, identify a material that forms at least a portion of the device body (e.g., a strap and/or watch housing). The haptic control unit may further, in step 804, select based on the identified material the defined range used in step 602 from among the plurality of defined ranges. In an embodiment, the plurality of ranges may be the defined ranges 117b stored in storage device 117. In an embodiment, each of the plurality of ranges may be associated with a different rigidity level. That is, softer materials may have different requirements for a quality coupling (neither too tight nor too lose) than harder materials. For instance, a more rigid material such as metal may allow a vibration to propagate more easily than a less rigid material such as leather. Thus, an authored haptic effect for a metal strap of a wearable electronic device may have a wider tolerance (i.e., wider range of tightness levels) than an authored haptic effect for a leather strap of another wearable electronic device. In another example, various wearable electronic devices may have respective materials that include a first material having a first rigidity level and a second material having a second rigidity level higher than the first rigidity level. The first material may be associated in a storage device with a first level of intensity, duration, or frequency. The second material may be stored in a storage device with a second level of intensity, duration, or frequency. The first level may have a higher intensity, longer duration, or lower frequency than that of the second level.

While the illustrated steps in FIG. 8 uses the material of the device body to select a defined range of tightness levels, the material may additionally or alternatively be used to select or otherwise determine (e.g., adjust) a haptic driving signal. For instance, if the defined haptic driving signal for an authored haptic effect was designed for an electronic watch with a leather strap, a haptic control unit for an electronic watch with metal links may adjust the defined haptic driving signal to have a decreased intensity or an increased frequency.

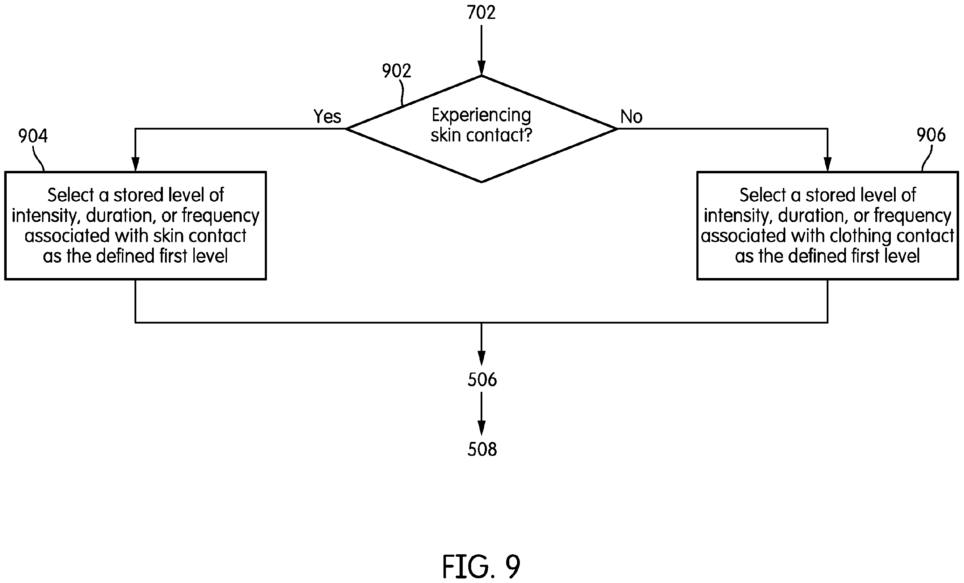

In an embodiment, an intensity, frequency, duration, and/or other characteristic of a haptic driving signal may be based on whether it is associated with skin contact, or instead with clothing contact. For instance, steps 604, 606, and 608 involve a defined first level of an intensity, duration, or frequency for a haptic driving signal. The defined first level may be used as a baseline level that can be adjusted based on whether the wearable electronic device for which the signal is generating a haptic effect is worn too tightly or too loosely. This baseline level may differ based on whether the wearable electronic device is in direct contact with a user's skin, or is instead in direct contact with the user's clothing (e.g., worn over a shirt cuff). Because clothing may make a haptic effect more difficult to perceive, the baseline level may be increased when the wearable electronic device is experiencing clothing contact. For instance, FIG. 9 illustrates a flow diagram of an embodiment in which the defined first level of intensity, duration, or frequency that is used in, e.g., steps 604, 606, and 608 is selected based on whether there the device body of a wearable electronic body is experiencing skin contact. In an embodiment, the steps of FIG. 9 may be preceded by step 702, in which a control unit may make a determination of whether a device body of a wearable electronic device is being worn. In response to a determination that the device body is being worn, the control unit may in step 902 determine whether the device body is experiencing skin contact. The determination may be performed by a skin contact sensor (e.g., a capacitive sensor). Such a sensor may, in an embodiment, be separate from the sensor used to measure the tightness level by which the device body is being worn.

As FIG. 9 further depicts, the control unit may, in response to a determination that the device body is being worn and experiencing skin contact, select a stored level of intensity, duration, or frequency associated with skin contact as being the defined first level (e.g., the defined first level used in steps 604, 606, or 608). In response to a determination that the device body is being worn and is not experiencing skin contact, the control unit may in step 906 select a stored level of intensity, duration, or frequency associated with clothing contact as the defined first level. The stored levels used in steps 904 and 906 may be stored in, e.g., haptic signal profiles 117a. As discussed above, because a haptic effect may be more difficult to perceive through clothing than if it were perceived directly on the skin, the baseline level for a haptic driving signal in steps 604, 606, or 608 may have to be increased if a wearable electronic device were experiencing clothing contact instead of a skin contact. Thus, in an embodiment, a level of intensity, duration, or frequency associated with clothing contact has a higher intensity, longer duration, or lower frequency than that of the stored level of intensity, duration, or frequency associated with skin contact.

In an embodiment, a wearable electronic device may have a plurality of haptic actuators (e.g., 113a, 113b) disposed on its device body, but if the device is worn too loosely, only a subset of the haptic actuators may be in contact with a user's body. Thus, in an embodiment, a control unit of the device may be configured to determine that the device body is being worn, and to determine which one or more haptic actuators of the plurality of haptic actuators are in contact with a user. The control unit may then select only from among the one or more haptic actuators that are determined to be in contact with the user to output the haptic effect.

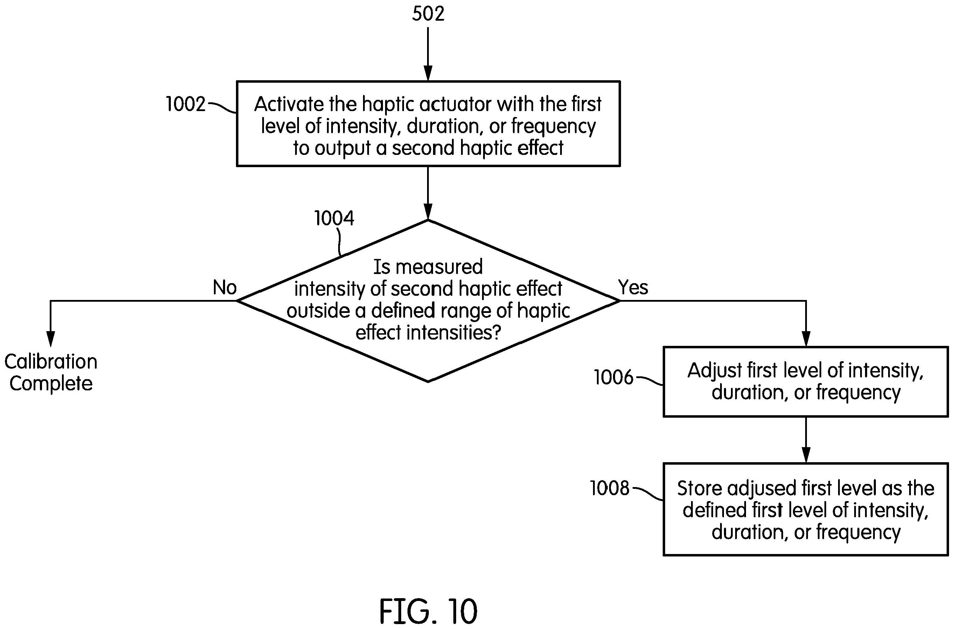

In an embodiment, the haptic control unit may be configured to perform a calibration process for a haptic actuator. The calibration process may involve adjusting a stored haptic driving signal. The adjusted signal may be stored as a separate signal, or may overwrite the currently stored haptic driving signal. FIG. 10 illustrates an example of a calibration process performed by a haptic control unit. The calibration process may involve a step 1002, in which the haptic control unit activates a haptic actuator with a first level of intensity, duration, or frequency to output a haptic effect. The haptic effect may be a separate haptic effect from that in step 510. The first level of intensity, duration, or frequency may have been defined in a profile (e.g., 117a) of a storage device. In step 1004, the control unit may determine whether a measured intensity of the haptic effect is outside a defined range of haptic effect intensities. For instance, the control unit may use an accelerometer to determine whether a measured acceleration of the haptic effect is outside a defined range of acceleration values. If the measured intensity is within the defined range, then adjustment of the haptic driving signal may be unnecessary, and the calibration process may come to an end. However, in response to a determination that the measured intensity is outside the defined range, the haptic control unit may in step 1006 adjust the first level of intensity, duration, or frequency. In step 1008, the control unit may store the adjusted first level as the defined first level of intensity, duration, or frequency. That is, the control unit may overwrite the previous first level in the stored profile with the adjusted first level.

While various embodiments have been described above, it should be understood that they have been presented only as illustrations and examples of the present invention, and not by way of limitation. It will be apparent to persons skilled in the relevant art that various changes in form and detail can be made therein without departing from the spirit and scope of the invention. Thus, the breadth and scope of the present invention should not be limited by any of the above-described exemplary embodiments, but should be defined only in accordance with the appended claims and their equivalents. It will also be understood that each feature of each embodiment discussed herein, and of each reference cited herein, can be used in combination with the features of any other embodiment. All patents and publications discussed herein are incorporated by reference herein in their entirety.

## Claims

1. A wearable electronic device, comprising:
a device body that is wearable at different tightness levels;
a sensor disposed on or within the device body, and configured, when the device body is being worn, to measure a parameter indicative of a tightness level by which the device body is being worn;
a haptic actuator disposed on or within the device body;
a control unit disposed on or within the device body and in communication with the sensor and with the haptic actuator, the control unit configured, when the device body is being worn,
to receive from the sensor a measurement of the parameter indicative of the tightness level by which the device body is being worn,
to determine the tightness level based on the measurement from the sensor,
to determine that a haptic effect is to be output at the device body,
to determine, based on the tightness level by which the device body is being worn, an intensity, duration, or frequency of a haptic driving signal for generating the haptic effect, and
to activate the haptic actuator with the haptic driving signal to output the haptic effect, the haptic driving signal having the intensity, duration, or frequency that is determined.

2. The wearable electronic device of claim 1, wherein at least a first portion of the device body is configured to fit around or substantially fit around a second portion of a user, wherein the sensor is at least one of a strain sensor and a pressure sensor, and wherein the parameter measured by the sensor is at least one of a strain in the first portion of the device body and a pressure experienced by the first portion of the device body.

3. The wearable electronic device of claim 2, wherein the first portion of the device body comprises a strap, the device body further comprising a housing that is coupled to the strap, wherein the housing houses the control unit, and wherein the housing and the strap together fit around the second portion of the user when the device body is being worn.

4. The wearable electronic device of claim 1, wherein at least a first portion of the device body is configured to fit around or substantially fit around a second portion of a user, wherein the sensor is disposed on a surface of the first portion of the device body and is at least one of a temperature sensor, a heart or pulse rate sensor, and a depth of field sensor, and wherein the parameter measured by the sensor is at least one of a strength of a heart rate signal or of a pulse rate signal, a temperature, a depth of field of another surface facing the sensor, and an amount of skin contact with the first portion.

5. The wearable electronic device of claim 1, wherein the control unit is configured to determine the at least one of the intensity, duration, or frequency of the haptic driving signal by:
determining i) whether the tightness level by which the device body is being worn is within a defined range of tightness levels, ii) whether the tightness level is above the defined range, or iii) whether the tightness level is below the defined range;
in response to a determination that the tightness level is within the defined range, determining the at least one of the intensity, duration, or frequency as having a defined first level,
in response to a determination that the tightness level is below the defined range, determining the at least one of the intensity, duration, or frequency as having a second level that has a higher intensity, longer duration, or lower frequency than that of the first level,
in response to a determination that the tightness level is above the defined range, determining the at least one of the intensity, duration, or frequency as having a third level that has a lower intensity, shorter duration, or higher frequency than that of the first level.

6. The wearable electronic device of claim 5, wherein the sensor is configured to measure the parameter even when the device body is not being worn,
wherein the control unit is configured to determine whether the device body is being worn by determining, based on the parameter measured by the sensor, whether the tightness level is at or above a defined tightness threshold, the defined tightness threshold being below the defined range of tightness levels, and
wherein the control unit determines that the haptic effect is to be output by the wearable electronic device only if the control unit has determined that the device body is being worn.

7. The wearable electronic device of claim 5, further comprising a storage device storing a profile that defines a plurality of defined ranges of tightness levels, and wherein the control unit is configured to identify a material that forms at least a portion of the device body, and is configured to select the defined range of tightness levels from among the plurality of defined ranges based on the material that is identified.

8. The wearable electronic device of claim 7, wherein the sensor is a first sensor, the wearable electronic device further comprising a second sensor that is a skin contact sensor configured to detect whether the device body is experiencing skin contact, and wherein the control unit is configured
in response to a determination that the device body is being worn and is experiencing skin contact, to select a stored level of intensity, duration, or frequency associated with skin contact as being the defined first level, and
in response to a determination that the device body is being worn and is not experiencing skin contact, to select a stored level of intensity, duration, or frequency associated with clothing contact as being the defined first level, wherein the stored level of intensity, duration, or frequency associated with clothing contact has a higher intensity, longer duration, or lower frequency than that of the stored level of intensity, duration, or frequency associated with skin contact.

9. The wearable electronic device of claim 5, wherein the control unit is further configured,
in response to a determination that the tightness level is within the defined range, to cause the haptic driving signal to have a first level of complexity by generating the haptic driving signal with a first number of pulses and a first duration between consecutive pulses, and
in response to a determination that the tightness level is below the defined range, to cause the haptic driving signal to have a second level of complexity lower than the first level by generating the haptic driving signal with a second number of pulses and a second duration between consecutive pulses, wherein the second number is less than the first number, and the second duration is longer than the first duration.

10. The wearable electronic device of claim 1, further comprising a storage device storing a profile that associates a plurality of levels of at least one of intensity, duration, or frequency of haptic driving signals with respective materials of device bodies, and is configured to determine the intensity, duration, or frequency of the haptic driving signal by identifying a material that forms at least a portion of the device body, and selecting one of the plurality of levels of intensity, duration, or frequency based on the material that is identified;
or
further comprising a storage device storing a profile that associates a plurality of levels of at least one of intensity, duration, or frequency of haptic driving signals with respective materials of device bodies, and is configured to determine the intensity, duration, or frequency of the haptic driving signal by identifying a material that forms at least a portion of the device body, and selecting one of the plurality of levels of intensity, duration, or frequency based on the material that is identified and wherein the plurality of materials includes a first material having a first rigidity level and a second material having a second rigidity level higher than the first rigidity level, and wherein the first material is associated with a first level of intensity, duration, or frequency that has a higher intensity, longer duration, or lower frequency than that of a second level of intensity, duration, or frequency associated with the second material.

11. The wearable electronic device of claim 1, wherein the haptic actuator is one of a plurality of haptic actuators disposed on the device body, and wherein the control unit is configured to determine that the device body is being worn, and to determine which one or more haptic actuators of the plurality of haptic actuators are in contact with a user, and is configured to select only from among the one or more haptic actuators that are determined to be in contact with the user to output the haptic effect.

12. The wearable electronic device of claim 1, further comprising a storage device that defines a first level of at least one of intensity, duration, or frequency at which to activate the haptic actuator, wherein the control unit is further configured to calibrate the haptic actuator by
activating the haptic actuator with the first level to output a second haptic effect,
determining whether a measured intensity of the second haptic effect is outside a defined range of haptic effect intensities, and
in response to a determination that the measured intensity is outside the defined range, adjust the first level of the at least one of intensity, duration, or frequency, and store the adjusted first level as the defined first level in the storage device.

13. A method of generating one or more haptic effects for a wearable electronic device having a device body that is wearable at different tightness levels, the method performed by a processor and comprising:
receiving, from a sensor disposed on or within the device body, a measurement of a parameter indicative of a tightness level by which the device body is being worn;
determining the tightness level based on the measurement from the sensor;
determining that a haptic effect is to be output at the device body;
determining, based on the tightness level by which the device body is being worn, an intensity, duration, or frequency of a haptic driving signal for generating the haptic effect; and
activating a haptic actuator disposed on or within the device body with the haptic driving signal to output the haptic effect, the haptic driving signal having the intensity, duration, or frequency that is determined.

14. The method of claim 13, wherein the step of determining the at least one of the intensity, duration, or frequency of the haptic driving signal comprises:
determining i) whether the tightness level by which the device body is being worn is within a defined range of tightness levels, ii) whether the tightness level is above the defined range, or iii) whether the tightness level is below the defined range, wherein the defined range of tightness levels is associated with a defined first level of intensity, duration, and frequency for the haptic driving signal.

15. The method of claim 13, wherein the tightness level is determined to be below the defined range, the method further comprising determining the at least one of the intensity, duration, or frequency as having a second level that has a higher intensity, longer duration, or lower frequency than that of the first level;
or
wherein the tightness level is determined to be above the defined range, the method further comprising determining the at least one of the intensity, duration, or frequency as having a third level that has a lower intensity, shorter duration, or higher frequency than that of the first level.

16. The method of claim 13, further comprising determining that the device body is being worn by determining, based on the parameter measured by the sensor, that the tightness level is at or above a defined tightness threshold, wherein the defined tightness threshold is below the defined range of tightness levels, and wherein the step of determining that the device body is being worn is performed before the step of determining that the haptic effect is to be output at the device body;
or
further comprising detecting whether the device body is experiencing skin contact or is instead receiving clothing contact, and wherein the step of determining the intensity, duration, or frequency of the haptic driving signal is based on whether the device body is experiencing skin contact or is instead receiving clothing contact.

17. The method of claim 13, wherein the step of determining the intensity, duration, or frequency of the haptic driving signal is based on a material that forms at least a portion of the device body.
